# EUROPÄISCHE PATENTSCHRIFT

(11) **EP 1 841 792 B1**
(45) Veröffentlichungstag und Bekanntmachung des Hinweises auf die Patenterteilung: **03.03.2010**
(21) Anmeldenummer: 06703804.2
(22) Anmeldetag: 20.01.2006
(51) Int. Cl.: C07K 14/765, A61K 47/48, A61K 31/04, A61P 35/00

(54) **VERFAHREN ZUR HERSTELLUNG VON ALBUMIN-KONJUGATEN MIT GYRASEHEMMERN**
METHOD FOR PRODUCING ALBUMIN CONJUGATES CONTAINING GYRASE INHIBITORS
PROCEDE DE PRODUCTION DE CONJUGUES D'ALBUMINE AVEC DES INHIBITEURS DE GYRASE

(30) Priorität: 21.01.2005 DE 102005002991
(43) Veröffentlichungstag der Anmeldung: 10.10.2007
(73) Patentinhaber: Albupharm Heidelberg GmbH & Co. KG, 69120 Heidelberg (DE)
(72) Erfinder: SINN, Hannsjörg, 69168 Wiesloch (DE); MÜLBAIER, Marcel, 69129 Heidelberg (DE)
(74) Vertreter: Dey, Michael
(86) Internationale Anmeldenummer: PCT/EP2006/000519
(87) Internationale Veröffentlichungsnummer: WO 2006/077146

(56) Entgegenhaltungen:
- WO-A-00/76551
- US-A1- 2003 203 917
- DATABASE MEDLINE [Online] US NATIONAL LIBRARY OF MEDICINE (NLM), BETHESDA, MD, US; Februar 1997 (1997-02), FISH D N ET AL: "The clinical pharmacokinetics of levofloxacin." XP002402238 Database accession no. NLM9068926 & CLINICAL PHARMACOKINETICS. FEB 1997, Bd. 32, Nr. 2, Februar 1997 (1997-02), Seiten 101-119, ISSN: 0312-5963
- ABOUL-FADL T ET AL: "SYNTHESIS AND IN VITRO INVESTIGATIONS OF NALIDIXIC ACID AMIDES OF AMINO ACID ESTERS AS PRODRUGS" PHARMAZIE, DIE, GOVI VERLAG, ESCHBORN, DE, Bd. 51, Nr. 1, Januar 1996 (1996-01), Seiten 30-33, XP008068690 ISSN: 0031-7144
- HOLTZAPPLE CAROL K; BUCKLEY SANDRA A; STANKER LARRY H: "Production and characterization of monoclonal antibodies against sarafloxacin and cross-reactivity studies of related fluoroquinolones" JOURNAL OF AGRICULTURAL AND FOOD CHEMISTRY, Bd. 45, Nr. 5, Mai 1997 (1997-05), Seiten 1984-1990, XP002402239
- HAMMER P; HEESCHEN W: "Antibody-capture immunoassay for the detection of enrofloxacin in raw milk" MILCHWISSENSCHAFT, Bd. 50, Nr. 9, 1995, Seiten 513-514, XP008068710 in der Anmeldung erwähnt
- KIDWAI M; MISRA P; KUMAR R: "The fluorinated quinolones" CURRENT PHARMACEUTICAL DESIGN, Bd. 4, Nr. 2, April 1998 (1998-04), Seiten 101-118, XP008068846
- KRATZ F: "Drug conjugates with albumin and transferrin" EXPERT OPINION ON THERAPEUTIC PATENTS, ASHLEY PUBLICATIONS, GB, Bd. 12, Nr. 3, 2002, Seiten 433-439, XP002337252 ISSN: 1354-3776
- STEHLE G ET AL: "The loading rate determines tumor targeting properties of methotrexate-albumin conjugates in rats" ANTI-CANCER DRUGS, RAPID COMMUNICATIONS, OXFORD, GB, Bd. 8, Nr. 7, August 1997 (1997-08), Seiten 677-685, XP009029047 ISSN: 0959-4973
- DAVIS M-T B ET AL: "A SIMPLE MODIFIED CARBODIIMIDE METHOD FOR CONJUGATION OF SMALL-MOLECULAR-WEIGHT COMPOUNDS TO IMMUNOGLOBULIN G WITH MINIMAL PROTEIN CROSSLINKING" ANALYTICAL BIOCHEMISTRY, ACADEMIC PRESS, NEW YORK, NY, US, Bd. 116, Nr. 2, 1981, Seiten 402-407, XP008061171 ISSN: 0003-2697
- INTERNET CITATION, [Online] 01 März 2002, XP001544035 Gefunden im Internet: <URL:www.roempp.com/prod/roempp.php> [gefunden am 2007-01-26]
- MUTSCHLER E.; SCHÄFER-KORING M.: 'Arzneimittelwirkung', 1996, WISSENSCHAFTLICHE VERLAGSGESELLSCHAFT MBH, STUTTGART Kapitel 9.2.1.6 Chinoloncarbonsäuren udn Analoge (Gyrasehemmer) * Seite 684 - Seite 687 *

## Beschreibung

Die vorliegende Erfindung betrifft Gyrasehemmer-Albumin-Konjugate, Arzneimittel umfassend Gyrasehemmer-Albumin-Konjugate, insbesondere zur Behandlung und/oder Prophylaxe von entzündlichen Prozessen und/oder Tumoren sowie Verfahren zu deren Herstellung.

Gyrasehemmer sind Antibiotika, die das Enzym DNA-Gyrase, das zu den Topoisomerasen 2 gehört, blockieren. Diese Enzyme falten die DNA-Helix an der Oberfläche von RNA-Kernen zu Schleifen, und die Schleifen werden gleichzeitig noch spiralig verdreht. Dies führt zu einer Anordnung mit minimalem Platzbedarf. Gleichzeitig wird durch diese Anordnung eine schnelle Replikation, Transkription und Rekombination der DNA begünstigt. Wird dieses Enzym durch einen Wirkstoff in seiner Funktion gehemmt oder gänzlich blockiert, ist eine sich teilende Zelle an einer zentralen, lebenswichtigen Stelle betroffen und nicht weiter lebensfähig.

Bisher wurden zur Therapie bakterieller Entzündungen verschiedene fluorhaltige Gyrasehemmer in ihrer niedermolekularen Form, wie z.B. Norfloxacin, Enoxacin oder Ciprofloxacin eingesetzt. Nachteilig bei den bisher angewandten Therapien unter Verwendung von Gyrasehemmern ist insbesondere die kurze Verweilzeit der eingesetzten Substanzen in der Zirkulation, sodass sie nur ein sehr schmales Zeitfenster für die Entfaltung ihrer Wirksamkeit aufweisen. Dies stellt das Grundproblem der medikamentösen Behandlung einer Erkrankung mit Gyrasehemmern dar, welches auf einer schnellen renalen und hepatobilären Ausscheidung beruht. So ist z.B. nach einer oralen Gabe von 400 mg Norfloxacin im Zeitraum von einer Stunde ein Maximalwert von 1 µg/ml Blut zu erwarten, was einer verfügbaren Menge von 1 bis 2 % der applizierten Gesamtdosis entspricht. Ein weiterer Nachteil der bisher eingesetzten Gyrasehemmer besteht darin, dass nur ein geringer Anteil des verabreichten Medikaments an den Zielort gelangt. Daraus resultieren unerwünschte Nebenwirkungen, die im Wesentlichen darauf basieren, dass der weitaus größte Anteil der Wirkstoffe von gesunden Organen aufgenommen wird und dort Nebenwirkungen , verursacht. Beobachtete Nebenwirkungen sind beispielsweise Beschwerden im Bereich des Magen-Darmtraktes, des Kreislaufs und des peripheren und zentralen Nervensystems wie erhöhte Erregbarkeit, Unruhe, Schlafstörungen, Verwirrtheit bis zu Halluzinationert und Krämpfen sowie Benommenheit bis zu psychotischen Zuständen. Weiterhin wurden Störungen der Haut, v.a. durch Photosensibilisierung sowie der Blutzellbildung, wie beispielsweise Thrombozytopenie, Leukopenie, etc. beobachtet.

P. Hammer und W. Heeschen (Milchwissenschaft 1995, 50(9), S. 513-514) offenbart ein Verfahren zur Herstellung eines Enrofloxacin-Rinderserumalbumin-Konjugates unter Verwendung von N-Hydroxysuccinimid, wasserfreiem Dimethylformamid sowie Dicyclohexylcarbodiimid (DCC). Dabei wird das Konjugat als Immunogen zur Antikörperherstellung verwendet, wobei der Antikörper, der für Enrofloxacin und Ciprofloxacin spezifisch ist, in einem indirekt-kompetitiven Immunoassay (ELISA) Verwendung findet. Ein wesentlicher Nachteil des in Hammer und Heeschen et al. offenbarten Konjugates liegt jedoch in dem aufgeführten hohen Molverhältnis von Enrofloxacin zu Rinderserumalbumin, welches etwa 25 : 1 beträgt, da bei einer solch hohen Beladung keinesfalls mehr von einem Protein in seiner natürlichen Form, sondern von einem massiv denaturierten Protein ausgegangen werden kann. Diese Denaturierung war jedoch das Ziel von Hammer und Heeschen, da nur mit einem solch denaturierten Protein die Immunisierung zur Antikörpergewinnung erfolgreich durchgeführt werden kann. Somit liegt ein denaturiertes, nicht mehr applikationsfähiges Produkt vor, welches für eine klinische Anwendung nicht in Betracht kommt. Es ist nicht mehr gewährleistet, dass der Wirkstoff direkt am Wirkort freigesetzt wird und somit nebenwirkungsfrei ist. Somit wird das von Hammer und Heeschen offenbarte Konjugat ausschließlich in vitro zum Nachweis der Gyrasehemmer Enrofloxacin und Ciprofloxacin in der Rohmilch von Kühen angewendet.

Eine Aufgabe der vorliegenden Erfindung war es deshalb, Gyrasehemmer in einer Form bereitzustellen, mit der die im Stand der Technik auftretenden Schwierigkeiten überwunden werden können und mit der insbesondere eine gezielte Aufnahme in bakteriell entzündetem Gewebe und/oder Tumorgewebe bei gleichzeitig langer Halbwertzeit, niedriger Dosierung und damit verbundenen geringeren Nebenwirkungen im Organismus erreicht werden kann.

Diese Aufgabe wird erfindungsgemäß gelöst durch Bereitstellung eines Gyrasehemmer-Albumin-Konjugats, welches einen Gyrasehemmer und Albumin umfasst, wobei das Molverhältnis Gyrasehemmer : Albumin 2 : 1 bis 0,1 : 1 beträgt, das Konjugat dadurch erhältlich ist, dass man einen Gyrasehemmer und Albumin in Gegenwart von N-(3-Dimethylaminopropyl)-N'-ethylcarbonyldiimid in einem organischen Lösungsmittel umsetzt, der Gyrasehemmer kovalent an Albumin gekoppelt ist und der Gyrasehemmer ausgewählt ist aus der Gruppe bestehend aus Chinoloncarbonsäure-Derivaten, 1,8-Naphthyridin-Derivaten, Pyridopyrimidincarbonsäure-Derivaten und/oder Cinnoloncarbonsäure-Derivaten.

Durch Kopplung von Gyrasehemmern an Proteine, insbesondere an Trägerproteine, werden die niedermolekularen Wirkstoffe, welche an sich schnell aus dem Körper eliminiert werden, vor den Ausscheidungs- bzw. Abfangmechanismen des Körpers verborgen und es wird eine lange Halbwertzeit und damit eine hohe Bioverfügbarkeit im Körper erreicht. Dadurch ist es möglich, geringe Mengen an Wirkstoff zu verabreichen und somit ggf. auftretende Nebenwirkungen wesentlich zu vermindern.

Toxische Effekte auf gesundes Gewebe oder Organe sind praktisch nicht zu beobachten, da normale, gesunde Zellen keine Veranlassung zur Proteinaufnahme haben. Im Gegensatz dazu werden Proteine und insbesondere Trägerproteine von mit entzündlichen Prozessen assoziierten Zellen oder von proliferierenden Tumorzellen aufgenommen und führen somit zu einer gezielten Wirkstoffanreicherung in diesen Zellen.

Bevorzugt wird als Albumin in den erfindungsgemäßen Konjugaten Serumalbumin und am meisten bevorzugt humanes Albumin bzw. humanes Serumalbumin (HSA) eingesetzt.

Grundsätzlich wird bevorzugt ein Protein verwendet, welches für den Patienten, für den das Konjugat vorgesehen ist, nativ ist. Unter einem nativen Protein ist ein Protein zu verstehen, das von der gleichen Spezies stammt wie diejenige Spezies, an die das Protein verabreicht wird. Das bedeutet beispielsweise, dass bei Verabreichung an Menschen humane Proteine, bei Verabreichung an Maus entsprechend Maus-Proteine, etc. eingesetzt werden.

Humanes Albumin ist ein körpereigenes, ubiquitär verteiltes und nicht immunogenes Protein. Es hat ein Molekulargewicht von etwa 68 kDa und wird somit nicht über die Niere ausgeschieden. Albumin macht ungefähr 60% der gesamten Plasma-Eiweißmenge aus. Es erfüllt im gesunden Organismus u.a. für viele Stoffe Transportfunktionen und dient im akuten Notfall als Reserve-Energieträger, der überall und jederzeit im Organismus verfügbar ist.

Unter physiologischen Bedingungen wird es vom gesunden Körpergewebe nicht aufgenommen. Im Gegensatz dazu, weisen mit entzündlichen, insbesondere mit bakteriellen Prozessen assoziierte Zellen sowie mit Tumoren, insbesondere soliden Tumoren, assoziierte Zellen einen hohen Umsatz an Proteinen, insbesondere an Plasmaproteinen, vorwiegend an Albumin auf. Über diesen Mechanismus wird das Albumin in den Zielzellen, für die das Protein als Energielieferant dient, abgebaut und der Wirkstoff wird freigesetzt. Dies bedeutet, dass durch die erfindungsgemäße Kopplung von Gyrasehemmern an Albumin, eine spezifische Aufnahme und damit eine Anreicherung des Wirkstoffes am Zielort erreicht werden kann. Durch diese zielgerichtete Anreicherung kann die Dosierung an Wirkstoff wesentlich reduziert werden, wodurch eine nebenwirkungsarme Behandlung gefördert wird, da die Wirkstoffe jetzt nur im Bereich des entzündlichen Prozesses oder in proliferierenden Tumorzellen durch enzymatische Spaltung des Proteins freigesetzt werden, während das Konjugat vom übrigen, gesunden Körpergewebe nicht aufgenommen wird. Ein weiterer Vorteil von Albumin liegt darin, dass es in klinisch verwendbarer Form jederzeit, auch in größeren Mengen, verfügbar ist.

Das biokinetische Verhalten und damit auch die biologische Halbwertzeit des erfindungsgemäßen Konjugates wird allein durch das Makromolekül Albumin bestimmt, nicht aber durch den niedermolekularen Gyrasehemmer. Damit fällt die anfänglich verfügbare Wirkstoffkonzentration erst nach ungefähr zwanzig Tagen auf etwa 50% des Ausgangswertes ab. Durch die lange biologische Halbwertszeit der Albuminkonjugate wird das bisher zeitlich sehr enge Zeitfenster der Wirkstoffverfügbarkeit wesentlich verbreitert, ohne dass weitere Nebenwirkungen auftreten. Folgemedikationen sind nur noch im Abstand von zwei bis drei Wochen erforderlich. Das erfindungsgemäß zur Bildung der Konjugate eingesetzte Albumin weist bevorzugt ein Molekulargewicht von ≥ 18.000 Da, besonders bevorzugt von ≥ 30.000 Da und insbesondere bevorzugt von ≥ 50.000 Da auf.

Die Kopplung des Gyrasehemmers an Albumin erfolgt bevorzugt ohne Einschränkung der biologischen Wirksamkeit des Wirkstoffs und ohne Verlust des natürlichen Charakters des als Träger genutzten Albumins. Unter einem Protein, das in seiner natürlichen Form vorliegt, versteht man insbesondere ein nicht denaturiertes, nicht alteriertes Protein, insbesondere ein Protein, das hinsichtlich seiner Eigenschaften, wie beispielsweise seiner Struktur, seiner physikochemischen Eigenschaften etc., nicht verändert ist. In den Konjugaten beträgt das Molverhältnis von Wirkstoff zu Albumin 2 : 1 bis 0,1 : 1, bevorzugt 1,5 : 1 bis 0,2 : 1, und insbesondere bevorzugt 1,1 : 1 bis 0,5 : 1. So zeigt Albumin bei einer 1 : 1 Beladung mit einem Gyrasehemmer immer noch biologisch aktives Verhalten.

Erfindungsgemäß erfolgt eine kovalente Kopplung des Wirkstoffs an das Trägerprotein Albumin. Weiterhin wird die kovalente Kopplung bevorzugt so gewählt, dass sie in pathologisch verändertem Geweben wieder spaltbar ist, sodass die biologische Wirksamkeit des ursprünglichen Wirkstoffs erhalten bleibt und genutzt werden kann. Die Spaltung erfolgt vorzugsweise enzymatisch. Dabei kann die Bindung an Albumin entweder direkt oder über einen Linker erfolgen.

Insbesondere ist eine direkte kovalente Kopplung des Wirkstoffes an das Trägerprotein bevorzugt. Eine direkte kovalente Kopplung des Wirkstoffes an den Träger bedeutet, dass der Wirkstoff durch eine Linker- bzw. Spacerfreie Bindung an das Transportprotein gebunden ist. Bevorzugt ist der Gyrasehemmer über eine Säureamidbindung, die aus einer Carboxylgruppe des Gyrasehemmers und einer Aminogruppe, bevorzugt einer Lysingruppe des Proteins gebildet wird, kovalent an Albumin gebunden.

Erfindungsgemäß geeignet hat sich der Einsatz von Gyrasehemmern erwiesen, die ausgewählt sind aus der Gruppe bestehend aus Chinoloncarbonsäure-Derivaten, 1,8-Naphtyridin-Derivaten, Pyridopyrimidincarbonsäure-Derivaten und/oder Cinnoloncarbonsäure-Derivaten.

In einer bevorzugten Ausführungsform der vorliegenden Erfindung sind geeignete Chinoloncarbonsäure-Derivate ausgewählt aus der Gruppe bestehend aus Ciprofloxacin, Enoxacin, Norfloxacin, Ofloxacin, Oxolinsäure, Sparfloxacin, Pefloxacin, Fleroxacin, Temafloxacin, Lomifloxacin, Ibafloxacin, Marbofloxacin, Danofloxacin, Moxifloxacin, Nadifloxacin, Enrofloxacin, Sarafloxacin und/oder Gatifloxacin, ist ein geeignetes 1,8-Naphtyridin-Derivat Nalidixinsäure, sind geeignete Pyridopyrimidincarbonsäure-Derivate ausgewählt aus der Gruppe bestehend aus Pipemidsäure und/oder Piromidsäure sowie geeignete Cinnoloncarbonsäure-Derivate ausgewählt aus der Gruppe bestehend aus Rosoxacin und/oder Cinoxacin.

Besonders bevorzugt sind gemäß der vorliegenden Erfindung die Gyrasehemmer Norfloxacin, Enoxacin und/oder Ciprofloxacin, insbesondere bevorzugt Norfloxacin an Albumin gebunden.

Besonders bevorzugt im Rahmen der vorliegenden Erfindung werden niedermolekulare Gyrasehemmer an das Transportprotein gebunden, da auf diese Weise allein das Makromolekül Albumin das biokinetische Verhalten, nicht aber der niedermolekulare Gyrasehemmer, bestimmt. Der erfindungsgemäß zur Bildung der Konjugate eingesetzte Gyrasehemmer weist bevorzugt ein Molekulargewicht von < 2000 Da, besonders bevorzugt < 1000 Da und insbesondere bevorzugt < 500 Da auf.

In einer bevorzugten Ausführungsform ist das erfindungsgemäße Konjugat Wirkstoff in einem Arzneimittel. Ein solches Arzneimittel weist insbesondere geringe Nebenwirkungen auf und kann beispielsweise auch ambulant verabreicht werden. Die Verabreichung erfolgt vorzugsweise intravenös.

Eine Dosiseinheit enthält vorzugsweise 1 bis 5 mg Wirkstoff Gyrasehemmer pro Kilogramm Körpergewicht pro 2 bis 3 Wochen und insbesondere 1 bis 2 mg Wirkstoff pro Kilogramm Körpergewicht pro 2 bis 3 Wochen. Die Dosis kann insbesondere niedriger als bei der herkömmlichen Therapie mit Gyrasehemmern gewählt werden und beträgt vorzugsweise ≤ 2 mg und besonders bevorzugt ≤ 1 mg Wirkstoff Gyrasehemmer pro Kilogramm Körpergewicht pro 2 bis 3 Wochen.

Während es möglich ist, eine Dosiseinheit pro Tag zu verabreichen, ist es aufgrund der langen biologischen Halbwertszeit der Konjugate im Körper auch möglich, längere Zeitintervalle zwischen der Verabreichung vorzusehen, sodass eine Dosiseinheit beispielsweise höchstens alle zwei Tage, besonders bevorzugt höchstens alle fünf Tage, insbesondere bevorzugt höchstens alle sieben Tage, und am meisten bevorzugt alle 14 bis 21 Tage verabreicht wird.

In einer bevorzugten Ausführungsform der vorliegenden Erfindung wird das Gyrasehemmer-Albumin-Konjugat zur Herstellung eines Arzneimittels zur Behandlung und/oder Prophylaxe von entzündlichen Prozessen, insbesondere von bakteriellen Entzündungen und/oder Tumoren, insbesondere soliden Tumoren, verwendet. Dabei wird das Arzneimittel bevorzugt an Säuger, Menschen und/oder Tiere und insbesondere an Menschen verabreicht.

Mit den erfindungsgemäßen Konjugaten können entzündliche Prozesse und Tumoren behandelt werden. Bakterielle Entzündungen, die erfindungsgemäß behandelt werden können, sind beispielsweise Infektionen der Niere, Harnwege, Geschlechtsorgane, Atemwege, des Bauchraums und der Haut. Dabei werden erfindungsgemäß Erreger wie beispielsweise Haemophilus influencae, Haemophilus ducreyi, Ulcus Molle, Legionellen, Brucellen, Chlamydien, Mycoplasmen/Ureaplasmen, Mycobakterien wie beispielsweise Mycobacterium tuberculosis und/oder gram-negative Stäbchenbakterien einschließlich vieler Pseudomonas aeruginosa-Stämme, gram-negative Kokken, Enteritis-Erreger wie beispielsweise Salmonellen, Shigellen, Yersinien und/oder Camphylobacter behandelt.

Weiterhin ist es möglich die erfindungsgemäßen Konjugate in der Kombinationstherapie einzusetzen, beispielsweise zusammen mit anderen antibakteriellen und/oder antitumoralen Mitteln. In einer solchen Kombinationstherapie reduzieren sich die Dosen der jeweiligen Komponenten weiter.

Ein weiterer Gegenstand der vorliegenden Erfindung ist ein Verfahren zur Herstellung eines Gyrasehemmer-Albumin-Konjugats, umfassend das Umsetzen eines Gyrasehemmers, bevorzugt eines niedermolekularen Gyrasehemmers mit Albumin,
wobei das Molverhältnis Gyrasehemmer : Albumin 2 : 1 bis 0,1 : 1 beträgt und wobei man einen Gyrasehemmer und Albumin in Gegenwart von N-(3-Dimethylaminopropyl)-N'-ethylcarbonyldiimid in einem organischen Lösungsmittel umsetzt.

Erfindungsgemäß erfolgt eine kovalente Kopplung des Gyrasehemmers an Albumin, ohne dessen natürlichen Charakter einzuschränken. Besonders vorteilhaft hat sich eine Kopplung erwiesen, bei der zunächst aus dem niedermolekularen Gyrasehemmer mittels eines Carbodiimids ein Succinimidylester gebildet wird und anschließend der Succinimidylester des Gyrasehemmers mit Albumin umgesetzt wird.

Für die Herstellung der erfindungsgemäß eingesetzten Konjugate ist eine effiziente kovalente Kopplung des Wirkstoffes an das Trägermolekül (also das Protein Albumin) von Bedeutung. Bei der Kopplung darf es insbesondere nicht zu einer unerwünschten Veränderung des Trägerproteins oder/und des Wirkstoffes kommen. Die bisher übliche Aktivierung Carboxylgruppen-haltiger organischer Verbindungen mit Dicyclohexylcarbodiimid (DCC) erfordert bei Raumtemperatur oder bei +4 °C mehr als 12 Stunden (P. Hammer und W. Heeschen (Milchwissenschaft 1995, 50(9), S. 513-514), DP 41 22 210 A1; EP 0 879 604 A1; EP 0 820 308). Bei diesem Verfahren bilden sich zudem bei der Aktivierung unlösliche Substanzen, die zum Teil bereits während der Aktivierung und zum Teil beim Eintragen des aktivierten Wirkstoffs in eine wässrige Proteinlösung ausfallen und, damit das Konjugat medizinisch verabreicht werden kann, durch zeitaufwändige und teure Filtrationsschritte zusätzlich zu der eigentlichen Produktreinigung abgetrennt werden müssen und nie 100%-ig sind (wegen der lipophilen Domänen im Albumin).

Es bestand deshalb der Bedarf, ein Verfahren zur Herstellung von Wirkstoff-Protein-Konjugaten bereitzustellen, bei dem diese Probleme nicht auftreten und bei dem insbesondere keine wasserunlöslichen Nebenprodukte gebildet werden.

Diese Aufgabe wurde erfindungsgemäß gelöst durch ein Verfahren zur Herstellung eines Konjugates, bei dem ein Gyrasehemmer und Albumin in Gegenwart von N-(3-Dimethylaminopropyl)-N'-ethylcarbodiimid (EDC) als Carbodiimid und N-Hydroxysuccinimid umgesetzt werden, wobei das Molverhältnis Gyrasehemmer zu Albumin 2 : 1 bis 0,1 : 1 beträgt.

Überraschenderweise wurde festgestellt, dass durch die Verwendung von EDC, insbesondere in Form des Hydrochlorids, eine Aktivierung der Carboxylgruppen-haltigen organischen Verbindung und eine Umsetzung mit einem Trägerprotein erreicht werden kann, ohne dass wasserunlösliche Nebenprodukte gebildet werden, die zeit- und kostenaufwändig abzutrennen wären. Durch dieses Verfahren werden Zwischenreinigungsschritte überflüssig und die Präparationszeit und damit auch die Herstellungskosten werden wesentlich reduziert. Weiterhin werden Probleme, die bei der Injektion des Konjuats in einen menschlichen oder tierischen Körper durch unlösliche Substanzen oder Nebenprodukte hervorgerufen werden, vermieden.

Die Aktivierung erfolgt bevorzugt bei einer Temperatur von 10 bis 100 °C, mehr bevorzugt von 20 bis 90 °C und noch mehr bevorzugt von 50 bis 75 °C für eine Reaktionszeit von 1 Minute bis 10 Stunden, mehr bevorzugt von 20 bis 50 Minuten. Die Umsetzung des aktivierten Wirkstoffes mit dem Trägerprotein erfolgt vorzugsweise bei einer Temperatur zwischen 10 und 50 °C, insbesondere zwischen 20 und 40 °C.

Bevorzugt wird die Aktivierung der Carboxyl-haltigen Verbindung, insbesondere von Norfloxacin mit EDC und N-Hydroxysuccinimid in einem organischen Lösungsmittel, bevorzugt in Dimethylsulfoxid (DMSO) durchgeführt. Weitere geeignete organische Lösungsmittel sind z.B. Dimethylacetamid oder Dioxan. Die Aktivierung wird vorzugsweise unter Ausschluss von Wasser durchgeführt, insbesondere in Gegenwart von ≤ 5 Gew.-% Wasser, mehr bevorzugt ≤ 1 Gew.-% Wasser und am meisten bevorzugt vollständig wasserfrei.

Ein wesentlicher Vorteil des erfindungsgemäßen Herstellungsverfahrens besteht darin, dass die eingesetzten Aktivierungsreagenzien, also EDC sowie N-Hydroxysuccinimid eine hohe Wasserlöslichkeit aufweisen. Dadurch können bei der Umsetzung nicht verbrauchte Kopplungsreagenzien auf einfache Weise aus dem gewonnen Produkt, beispielsweise durch Waschen mit Wasser entfernt werden. Im Gegensatz dazu verbleibt bei den im Stand der Technik verwendeten Kopplungsreagenzien, beispielsweise bei der Verwendung von Di-cyclohexyl-carbodiimid (DCC) ein nicht auftrennbarer Rest an Kopplungsreagenz im Konjugat. So ist bei einem Gyrasehemmer-Albumin-Konjugat bei Verwendung von DCC ein nicht abtrennbarer Rest von etwa 13 bis 15 Gew.-% an DCC im Konjugat zu beobachten, der wahrscheinlich an eine lipophile Domäne im Albumin gebunden ist. Dieser Rest kann nur mit Hilfe von HPLC nachgewiesen und nur unter erheblichem Aufwand präparativ abgetrennt werden.

Wird insbesondere noch mit einem hohen Überschuss an DCC im Verhältnis zum zu koppelnden Wirkstoff, wie es in der Veröffentlichung von P. Hammer und W. Heeschen (siehe oben) beschrieben ist, gearbeitet, nämlich einem Molverhältnis DCC : Wirkstoff von etwa 10 : 1, so ist eine vollständige Abtrennung des Proteins mittels Dialyse oder Ultrafiltration nicht mehr möglich. Dieses am Protein haftende, immer noch reaktive DCC führt jedoch im Laufe der Zeit durch intramolekulares und intermolekulares Crosslinking zu einer weiter fortschreitenden Alteration des Proteins, wodurch sich die Eigenschaften des Trägerproteins zeitabhängig verändern. Daher kommt für ein so hergestelltes Konjugat keine klinische Anwendung in Betracht.

Ein weiterer bevorzugter Aspekt der Erfindung betrifft ein optimiertes Herstellungsverfahren eines erfindungsgemäßen Konjugats umfassend das Umsetzen eines Gyrasehemmers mit Albumin durch eine direkte kovalente Kopplung, **dadurch gekennzeichnet, dass** man einen Gyrasehemmer und Albumin in Gegenwart eines Carbodiimids, bevorzugt in Gegenwart von N-(3-Dimethylaminopropyl)-N'-ethyl-carbodiimid ohne N-Hydroxysuccinimid oder N-Hydroxysuccinamid oder ein beliebiges weiteres Aktivierungsreagenz, umsetzt.

In einer bevorzugten Ausführungsform handelt es sich bei dem Gyrasehemmer bevorzugt um einen Gyrasehemmer, der ausgewählt ist aus der Gruppe bestehend aus Chinoloncarbonsäure-Derivaten, 1,8-Naphtyridin-Derivaten, Pyridopyrimidincarbonsäure-Derivaten und/oder Cinnoloncarbonsäure-Derivaten. Besonders bevorzugt sind die Chinoloncarbonsäure-Derivate, ausgewählt aus der Gruppe bestehend aus Ciprofloxacin, Enoxacin, Norfloxacin, Ofloxacin, Oxolinsäure, Sparfloxacin, Pefloxacin, Fleroxacin, - Temafloxacin, Lomifloxacin, Ibafloxacin, Marbofloxacin, Danofloxacin, Moxifloxacin, Nadifloxacin, Enrofloxacin, Sarafloxacin und/oder Gatifloxacin. Das 1,8-Naphthyridin-Derivat ist bevorzugt Nalidixinsäure und die Pyridopyrimidincarbonsäure-Derivate sind bevorzugt ausgewählt aus der Gruppe bestehend aus Pipemidsäure und/oder Piromidsäure und die Cinnoloncarbonsäure-Derivate sind bevorzugt ausgewählt aus der Gruppe bestehend aus Rosoxacin und/oder Cinoxacin. Besonders bevorzugt handelt es sich bei den Gyrasehemmern um Norfloxacin, Enoxacin und/oder Ciprofloxacin, und insbesondere bevorzugt um Norfloxacin. Beim Protein handelt es sich bevorzugt um Albumin.

Überraschenderweise wurde festgestellt, dass sich das optimierte Verfahren, welches ohne N-Hydroxysuccinimid oder N-Hydroxysuccinamid oder andere zusätzliche Aktivierungsreagenzien arbeitet, bei der Reinigungsprozedur positiv auf die Vereinfachung der Herstellung auswirkt. Durch die Verwendung von EDC zur Aktivierung ohne den Zusatz von N-Hydroxysuccinimid (HSI) beträgt die Aktivierungszeit von Norfloxacin nur noch wesentlich weniger als die bei Verwendung von HSI benötigten 30 Minuten. Ein weiterer Vorteil des optimierten Verfahrens liegt darin, dass nach Zugabe des aktivierten Wirkstoffs zu dem vorgelegten Albumin ohne N-Hydroxysuccinimid eine direkte Kontrolle der Kopplungseffizienz erfolgen kann. Bei Verwendung von N-Hydroxysuccinimid besitzt dieses bei der HPLC bei einer Einstellung der UV-Messzelle auf 280 nm ebenfalls eine hohe UV-Absorption und stört oder erschwert durch seine Retentionszeit von etwa 11,5 Minuten, bei der auch andere niedermolekulare Verbindungen erscheinen, eine direkte Bestimmung der Kopplungsausbeute. Dies bedeutet, dass in vielen Fällen eine Ausbeutebestimmung erst am Ende der Reinigung des Konjugats möglich ist. Durch das optimierte Verfahren ohne Einsatz von N-Hydroxysuccinimid kann dieser Faktor nun ausgeschlossen werden. Dies ist auch für die Produktsicherheit von großem Vorteil. Ein weiterer Vorteil des optimierten Verfahrens liegt darin, dass die Kopplungsausbeute überraschenderweise durchschnittlich bei 98 bis 99 % liegt.

Somit sind die Gesamtkosten des jeweiligen Konjugats durch diese Vereinfachung der Herstellung deutlich gesenkt.

Die mit den erfindungsgemäßen Verfahren hergestellten Konjugate können aufgrund ihrer hohen Reinheit für zahlreiche Verwendungen und insbesondere für eine intravenöse Verabreichung vorgesehen werden. Somit können solche Konjugate, beispielsweise bei Verwendung eines Gyrasehemmers mit entzündungshemmender Wirkung bzw. mit Antitumorwirkung vorteilhafterweise zur Herstellung von Arzneimitteln zur Prophylaxe und/oder zur Behandlung entzündlicher Prozesse, insbesondere zur Herstellung eines Arzneimittels zur Behandlung von bakteriellen Entzündungen oder zur Behandlung solider Tumoren eingesetzt werden.

Die Erfindung wird durch das folgende Beispiel und die beigefügten Figuren weiter erläutert.

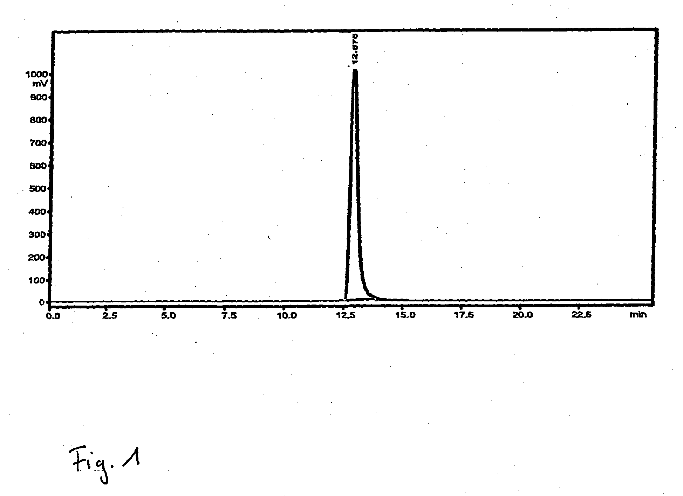

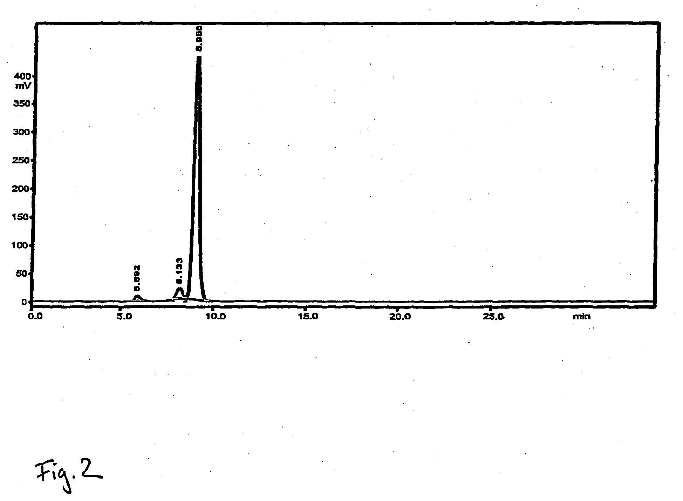

Figur 1 zeigt ein HPLC Chromatogramm von Norfloxacin alleine, und Figur 2 zeigt das Chromatogramm des gemäß Beispiel 1 hergestellten Norfloxacin-HSA-Konjugats.

### Beispiel 1

### Norfloxacin-HSA-Konjugat

### Ausgangsstoffe:

### Norfloxacin (SIGMA-ALDRICH, Taufkirchen), N-(3-Dimethylaminopropyl)-N'-ethylcarbodiimid Hydrochlorid (SIGMA-ALDRICH, Taufkirchen), N-Hydroxysuccinimid (SIGMA ALDRICH, Taufkirchen) und Albumin (Göricke, Dessau).

Anstelle des Norfloxacins können auch andere carboxygruppenhaltige Gyrasehemmer, die ausgewählt sind aus der Gruppe bestehend aus Chinoloncarbonsäure-Derivaten, 1,8-Naphthyridin-Derivaten, Pyridopyrimidincarbonsäure-Derivaten und/oder Cinnoloncarbonsäure-Derivaten, zur Konjugation mit Albumin eingesetzt werden.

### Standardansatz im Labormaßstab:

Etwa 12,2 mg Norfloxacin (MG 319,3) werden zusammen mit etwa 14,6 mg N-(3-Dimethylaminopropyl)-N'-ethylcarbodiimid Hydrochlorid (MG 191,71, Molverhältnis 1:2) und etwa 44 mg N-Hydroxysuccinimid (MG 115,9, Molverhältnis 1:10) in einem Reagenzglas mit NS 14,5 Schliff und Stopfen vorgelegt. Nach der Zugabe von 1 mL Dimethylsulfoxid (DMSO) oder ähnlichen polaren Lösungsmitteln wird die Reaktionsmischung in ein auf 65 °C vorgeheiztes Wasserbad eingebracht. Nach einer Reaktionszeit von etwa 30 Minuten liegt eine klare, farblose Lösung von Norfloxacinsuccinimidylester vor, die nach Abkühlung auf Raumtemperatur sehr langsam in eine 5%ige Albuminlösung eingetragen wird. An der Einlaufstelle bildet sich kurzzeitig eine Trübung, die sich jedoch schnell wieder auflöst.
Das Kontrollchromatogramm kann direkt im Anschluss an die Kopplung angefertigt werden.
Die Abtrennung der im Endprodukt unerwünschten Begleitstoffe DMSO, NHS, N-(3-Dimethylaminopropyl)-N'-ethyl-Harnstoff und nicht kovalent gebundenes Norfloxacin erfolgt durch Ultrafiltration (YM 30, Millipore).

### Qualitätskontrolle (HPLC/SEC):

| | |
|---|---|
| Vorsäule: | Reprosil 200 SEC 5 x 4 mm, 5 µm (Dr. Maisch GmbH) |
| Säule: | Reprosil 200 SEC 300 x 4,6 mm, 5 µm (Dr. Maisch GmbH) |
| Laufmittel: | 0,18 M Na₂HPO₄; pH 7,4; 5% Methanol |
| Fluß: | 0,3 mL/min |
| Druck: | etwa 50 bar |
| UV-vis: | 280 nm |

### Retentionszeiten:

| | |
|---|---|
| oligomere Albuminfraktion | 5,90 min |
| dimere Albumin-Fraktion | 8,14 min |
| monomere Albumin-Fraktion | 8,96 min |
| freies Norfloxacin | 12,88 min |

**Chromatogramme:** siehe Figur 1 und 2

## Patentansprüche

1. Gyrasehemmer-Albumin-Konjugat, umfassend einen Gyrasehemmer und Albumin,
**dadurch gekennzeichnet,**
**dass** das Molverhältnis Gyrasehemmer zu Albumin 2 : 1 bis 0,1 : 1 beträgt, wobei das Konjugat **dadurch** erhältlich ist, dass man einen Gyrasehemmer und Albumin in Gegenwart von N-(3-Dimethylaminopropyl)-N'-ethylcarbonyldiimid in einem organischen Lösungsmittel umsetzt, wobei
der Gyrasehemmer kovalent an Albumin gekoppelt ist, und wobei
der Gyrasehemmer ausgewählt ist aus der Gruppe bestehend aus Chinoloncarbonsäure-Derivaten, 1,8-Naphthyridin-Derivaten, Pyridopyrimidincarbonsäure-Derivaten und/oder Cinnoloncarbonsäure-Derivaten.

2. Gyrasehemmer-Albumin-Konjugat nach Anspruch 1,
**dadurch gekennzeichnet,**
**dass** das Albumin humanes Serum-Albumin ist.

3. Gyrasehemmer-Albumin-Konjugat nach einem der Ansprüche 1 oder 2,
**dadurch gekennzeichnet,**
**dass** Albumin in nativer Form vorliegt.

4. Gyrasehemmer-Albumin-Konjugat nach Anspruch 3,
**dadurch gekennzeichnet,**
**dass** die kovalente Kopplung unter pathologischen Bedingungen gespalten wird.

5. Gyrasehemmer-Albumin-Konjugat nach Anspruch 4,
**dadurch gekennzeichnet,**
**dass** die Spaltung enzymatisch erfolgt.

6. Gyrasehemmer-Albumin-Konjugat nach einem der Ansprüche 1 bis 5,
**dadurch gekennzeichnet,**
**dass** Albumin in seiner natürlichen Form vorliegt.

7. Gyrasehemmer-Albumin-Konjugat nach Anspruch 6,
**dadurch gekennzeichnet,**
**dass** die Chinoloncarbonsäure-Derivate ausgewählt sind aus der Gruppe bestehend aus Ciprofloxacin, Enoxacin, Norfloxacin, Ofloxacin, Oxolinsäure, Sparfloxacin, Pefloxacin, Fleroxacin, Temafloxacin, Lomifloxacin, Ibafloxacin, Marbofloxacin, Danofloxacin, Moxifloxacin, Nadifloxacin, Enrofloxacin, Sarafloxacin und/oder Gatifloxacin,
das 1,8-Naphthyridin-Derivat Nalidixinsäure ist, die Pyridopyrimidincarbonsäure-Derviate ausgewählt sind aus der Gruppe bestehend aus Pipemidsäure und/oder Piromidsäure und die Cinnoloncarbonsäure-Derivate ausgewählt sind aus der Gruppe bestehend aus Rosoxacin und/oder Cinoxacin.

8. Gyrasehemmer-Albumin-Konjugat nach Anspruch 7,
**dadurch gekennzeichnet,**
**dass** der Gyrasehemmer ausgewählt ist aus der Gruppe bestehend aus Norfloxacin, Enoxacin und/oder Ciprofloxacin ist.

9. Gyrasehemmer-Albumin-Konjugat nach Anspruch 8,
**dadurch gekennzeichnet,**
**dass** der Gyrasehemmer Norfloxacin ist.

10. Gyrasehemmer-Albumin-Konjugat nach Anspruch 1,
**dadurch gekennzeichnet,**
**dass** das Molverhältnis Gyrasehemmer zu Albumin 1,1 : 1 bis 0,5 : 1 beträgt.

11. Gyrasehemmer-Albumin-Konjugat nach Anspruch 1 oder 10,
**dadurch gekennzeichnet,**
**dass** das Molverhältnis Gyrasehemmer zu Albumin 1,1 : 1 bis 0,9 : 1 beträgt.

12. Arzneimittel, umfassend als Wirkstoff ein Gyrasehemmer-Albumin-Konjugat nach einem der Ansprüche 1 bis 11.

13. Verwendung eines Gyrasehemmer-Albumin-Konjugats nach einem der Ansprüche 1 bis 11 zur Herstellung eines Arzneimittels zur Behandlung und/oder Prophylaxe von entzündlichen Prozessen und/oder Tumoren.

14. Verwendung nach Anspruch 13,
**dadurch gekennzeichnet,**
**dass** es sich bei den entzündlichen Prozessen um bakterielle Entzündungen oder bei den Tumoren um solide Tumoren handelt.

15. Verfahren zur Herstellung eines Gyrasehemmer-Albumin-Konjugats nach Anspruch 1 umfassend das Umsetzen eines Gyrasehemmers mit Albumin,
**dadurch gekennzeichnet,**
**dass** das Molverhältnis Gyrasehemmer zu Albumin 2 : 1 bis 0,1 : 1 beträgt und man einen Gyrasehemmer und Albumin in Gegenwart von N-(3-Dimethylaminopropyl)-N'-ethyl-carbonyldiimid in einem organischen Lösungsmittel umsetzt.

16. Verfahren nach Anspruch 15,
**dadurch gekennzeichnet,**
**dass** zunächst aus dem Gyrasehemmer ein Succinimidylester mittels N-(3-Dimethylaminopropyl)-N'-ethyl-carbonyldiimid und N-Hydroxysuccinimid gebildet wird und anschließend der Succinimidylester des Gyrasehemmers mit Albumin umgesetzt wird.

17. Verfahren zur Herstellung eines Gyrasehemmer-Albumin-Konjugats nach Anspruch 1, umfassend das Umsetzen eines Gyrasehemmers mit Albumin,
**dadurch gekennzeichnet,**
**dass** das Molverhältnis Gyrasehemmer zu Albumin 2 : 1 bis 0,1 : 1 beträgt und man einen Gyrasehemmer und Albumin in Gegenwart von N-(3-Dimethylaminopropyl)-N'-ethyl-carbonyldiimid als Aktivierungsreagenz ohne N-Hydroxysuccinimid und/oder ohne N-Hydroxysuccinamid in einem organischen Lösungsmittel umsetzt.

18. Verfahren zur Herstellung eines Gyrasehemmer-Albumin-Konjugats nach Anspruch 1, umfassend das Umsetzen eines Gyrasehemmers mit Albumin,
**dadurch gekennzeichnet,**
**dass** man einen Gyrasehemmer und Albumin in Gegenwart von N-(3-Dimethylaminopropyl)-N'-ethylcarbodiimid als Aktivierungsreagenz ohne weiteres Aktivierungsreagenz in einem organischen Lösungsmittel umsetzt.

19. Verfahren nach einem der Ansprüche 15 bis 18,
**dadurch gekennzeichnet,**
**dass** das organische Lösungsmittel wasserfreies organisches Lösungsmittel ist.

20. Verfahren nach einem der Ansprüche 17 bis 19,
**dadurch gekennzeichnet,**
**dass** der Gyrasehemmer mit N-(3-Dimethylaminopropyl)-N'-ethylcarbodiimid umgesetzt, durch Erwärmung aktiviert und anschließend der Gyrasehemmer mit Albumin umgesetzt wird.

21. Verfahren nach einem der Ansprüche 15 bis 20,
**dadurch gekennzeichnet,**
**dass** es sich bei Albumin um humanes Serum-Albumin handelt.

## Claims

1. Gyrase inhibitor-albumin conjugate comprising a gyrase inhibitor and albumin,
**characterised in**
**that** the molar ratio of the gyrase inhibitor to albumin is 2:1 to 0.1:1, wherein the conjugate is obtained by reacting a gyrase inhibitor and albumin in the presence of N-(3-dimethyl aminopropyl)-N'-ethyl-carbonyl diimide in an organic solvent,
wherein
the gyrase inhibitor is covalently coupled to albumin, and wherein the gyrase inhibitor is selected from the group comprising quinolone carboxylic acid derivatives, 1,8-naphthyridine derivatives, pyridopyrimidine carboxylic acid derivatives and/or cinnolone carboxylic acid derivatives.

2. Gyrase inhibitor-albumin conjugate according to Claim 1,
**characterised in**
**that** the albumin is human serum albumin.

3. Gyrase inhibitor-albumin conjugate according to either Claim 1 or Claim 2, **characterised in**
**that** the albumin is present in native form.

4. Gyrase inhibitor-albumin conjugate according to Claim 3,
**characterised in**
**that** the covalent coupling is cleaved under pathological conditions.

5. Gyrase inhibitor-albumin conjugate according to Claim 4,
**characterised in**
**that** the cleavage takes place enzymatically.

6. Gyrase inhibitor-albumin conjugate according to any one of Claims 1 to 5, **characterised in**
**that** albumin is present in its natural form.

7. Gyrase inhibitor-albumin conjugate according to Claim 6,
**characterised in**
**that** the quinolone carboxylic acid derivatives are selected from the group comprising ciprofloxacin, enoxacin, norfloxacin, ofloxacin, oxolinic acid, sparfloxacin, pefloxacin, fleroxacin, temafloxacin, lomifloxacin, ibafloxacin, marbofloxacin, danofloxacin, moxifloxacin, nadifloxacin, enrofloxacin, sarafloxacin and/or gatifloxacin, the 1,8-naphthyridine derivative is nalidixic acid, the pyridopyrimidine carboxylic acid derivatives are selected from the group comprising pipemidic acid and/or piromidic acid and the cinnolone carboxylic acid derivatives are selected from the group comprising rosoxacin and cinoxacin.

8. Gyrase inhibitor-albumin conjugate according to Claim 7,
**characterised in**
**that** the gyrase inhibitor is selected from the group comprising norfloxacin, enoxacin and/or ciprofloxacin.

9. Gyrase inhibitor-albumin conjugate according to Claim 8,
**characterised in**
**that** the gyrase inhibitor is norfloxacin.

10. Gyrase inhibitor-albumin conjugate according to Claim 1,
**characterised in**
**that** the molar ratio of gyrase inhibitor to albumin is 1.1:1 to 0.5:1.

11. Gyrase inhibitor-albumin conjugate according to Claim 1 or 10,
**characterised in**
**that** the molar ratio of gyrase inhibitor to albumin is 1.1:1 to 0.9:1.

12. A medicament, comprising as an active ingredient a gyrase inhibitor-albumin conjugate according to any one of Claims 1 to 11.

13. Use of a gyrase inhibitor-albumin conjugate according to any one of Claims 1 to 11 for the manufacture of a medicament for the treatment and/or prophylaxis of inflammatory processes and/or tumours.

14. Use according to Claim 13,
**characterised in**
**that** the inflammatory processes are bacterial inflammations or the tumours are solid tumours.

15. A method for the preparation of a gyrase inhibitor-albumin conjugate comprising reacting a gyrase inhibitor with albumin,
**characterised in**
**that** the molar ratio of gyrase inhibitor to albumin is 2:1 to 0.1:1 and a gyrase inhibitor and albumin are reacted in the presence of N-(3-dimethylaminopropyl)-N'-ethyl-carbonyl diimide in an organic solvent.

16. A method according to Claim 15,
**characterised in**
**that** firstly a succinimidyl ester is formed from the gyrase inhibitor by means of N-(3-dimethylaminopropyl)-N'-ethyl-carbonyl diimide and N-hydroxy-succinimide, and subsequently the succinimidyl ester of the gyrase inhibitor is reacted with albumin.

17. A method for the preparation of a gyrase inhibitor-albumin conjugate according to Claim 1, comprising reacting a gyrase inhibitor with albumin,
**characterised in**
**that** the molar ratio of gyrase inhibitor to albumin is 2:1 to 0.1:1 and a gyrase inhibitor and albumin are reacted in the presence of N-(3-dimethylamino-propyl)-N'-ethyl-carbonyl diimide as an activation reagent without N-hydroxysuccinimide and/or without N-hydroxysuccinamide in an organic solvent.

18. A method for the preparation of a gyrase inhibitor-albumin conjugate comprising reacting a gyrase inhibitor with albumin,
**characterised in**
**that** a gyrase inhibitor and albumin are reacted in an organic solvent in the presence of N-(3-dimethylaminopropyl)-N'-ethylcarbodiimide as the activation reagent without a further activation reagent.

19. A method according to any one of Claims 15 to 18,
**characterised in**
**that** the organic solvent is an anhydrous organic solvent.

20. A method according to any one of Claims 17 to 19,
**characterised in**
**that** the gyrase inhibitor is reacted with N-(3-dimethylaminopropyl)-N'-ethyl-carbodiimide, activated by heating and subsequently the gyrase inhibitor is reacted with the albumin.

21. A method according to any one of Claims 15 to 20,
**characterised in**
**that** the albumin is human serum albumin.

## Revendications

1. Conjugué d'inhibiteur de la gyrase et d'albumine, comprenant un inhibiteur de la gyrase et de l'albumine, **caractérisé en ce que** le rapport molaire de l'inhibiteur de la gyrase sur l'albumine se situe dans la plage de 2:1 à 0,1:1, le conjugué pouvant être obtenu en faisant réagir un inhibiteur de la gyrase et de l'albumine en présence de *N*-(3-diméthylaminopropyl)-*N'*-éthylcarbonyldiimide dans un solvant organique, l'inhibiteur de la gyrase étant couplé de manière covalente à l'albumine et l'inhibiteur de la gyrase étant choisi dans le groupe constitué par des dérivés de l'acide quinolone carboxylique, des dérivés de 1,8-naphtyridine, des dérivés de l'acide pyridopyrimidine carboxylique et/ou des dérivés de l'acide cinnolone carboxylique.

2. Conjugué d'inhibiteur de la gyrase et d'albumine selon la revendication 1, **caractérisé en ce que** l'albumine est la sérum albumine humaine.

3. Conjugué d'inhibiteur de la gyrase et d'albumine selon la revendication 1 ou la revendication 2, **caractérisé en ce que** l'albumine est présente sous forme native.

4. Conjugué d'inhibiteur de la gyrase et d'albumine selon la revendication 3, **caractérisé en ce que** le couplage covalent est dissocié en présence de conditions pathologiques.

5. Conjugué d'inhibiteur de la gyrase et d'albumine selon la revendication 4, **caractérisé en ce que** la dissociation se fait par voie enzymatique.

6. Conjugué d'inhibiteur de la gyrase et d'albumine selon l'une des revendications 1 à 5, **caractérisé en ce que** l'albumine est présente sous sa forme naturelle.

7. Conjugué d'inhibiteur de la gyrase et d'albumine selon la revendication 6, **caractérisé en ce que** les dérivés de l'acide quinolone carboxylique sont choisis dans le groupe constitué par la ciprofloxacine, l'énoxacine, la norfloxacine, l'ofloxacine, l'acide oxolinique, la sparfloxacine, la péfloxacine, la fléroxacine, la témafloxacine, la lomifloxacine, l'ibafloxacine, la marbofloxacine, la danofloxacine, la moxifloxacine, la nadifloxacine, l'enrofloxacine, la sarafloxacine et/ou la gatifloxacine ; le dérivé de 1,8-naphtyridine est l'acide nalidixique ; les dérivés de l'acide pyridopyrimidine carboxylique sont choisis dans le groupe constitué par l'acide pipémidique et/ou l'acide piromidique ; et les dérivés de l'acide cinnolone carboxylique sont choisis dans le groupe constitué par la rosoxacine et/ou la cinoxacine.

8. Conjugué d'inhibiteur de la gyrase et d'albumine selon la revendication 7, **caractérisé en ce que** l'inhibiteur de la gyrase est choisi dans le groupe constitué par la norfloxacine, l'énoxacine et/ou la ciprofloxacine.

9. Conjugué d'inhibiteur de la gyrase et d'albumine selon la revendication 8, **caractérisé en ce que** l'inhibiteur de la gyrase est la norfloxacine.

10. Conjugué d'inhibiteur de la gyrase et d'albumine selon la revendication 1, **caractérisé en ce que** le rapport molaire de l'inhibiteur de la gyrase sur l'albumine se situe dans la plage de 1,1:1 à 0,5:1.

11. Conjugué d'inhibiteur de la gyrase et d'albumine selon la revendication 1 ou la revendication 10, **caractérisé en ce que** le rapport molaire de l'inhibiteur de la gyrase sur l'albumine se situe dans la plage de 1,1:1 à 0,9:1.

12. Médicament comprenant comme principe actif un conjugué d'inhibiteur de la gyrase et d'albumine selon l'une des revendications 1 à 11.

13. Utilisation d'un conjugué d'inhibiteur de la gyrase et d'albumine selon l'une des revendications 1 à 11 pour fabriquer un médicament destiné au traitement et/ou à la prévention de processus inflammatoires et/ou de tumeurs.

14. Utilisation selon la revendication 13, **caractérisée en ce que** les processus inflammatoires sont des inflammations bactériennes ou **en ce que** les tumeurs sont des tumeurs solides.

15. Procédé de préparation d'un conjugué d'inhibiteur de la gyrase et d'albumine selon la revendication 1, comprenant la réaction d'un inhibiteur de la gyrase avec de l'albumine, **caractérisé en ce que** le rapport molaire de l'inhibiteur de la gyrase sur l'albumine se situe dans la plage de 2:1 à 0,1:1 et **en ce que** l'on fait réagir un inhibiteur de la gyrase et de l'albumine en présence de *N*-(3-diméthylaminopropyl)-*N'*-éthylcarbonyldiimide dans un solvant organique.

16. Procédé selon la revendication 15, **caractérisé en ce que** l'on commence par former un ester succinimidylique de l'inhibiteur de la gyrase au moyen de *N*-(3-diméthylaminopropyl)-*N*-éthylcarbonyldiimide et de *N*-hydroxysuccinimide et ensuite on fait réagir l'ester succinimidylique de l'inhibiteur de la gyrase avec de l'albumine.

17. Procédé de préparation d'un conjugué d'inhibiteur de la gyrase et d'albumine selon la revendication 1, comprenant la réaction d'un inhibiteur de la gyrase avec de l'albumine, **caractérisé en ce que** le rapport molaire de l'inhibiteur de la gyrase sur l'albumine se situe dans la plage de 2:1 à 0,1:1 et **en ce que** l'on fait réagir un inhibiteur de la gyrase et de l'albumine en présence de *N*-(3-diméthylaminopropyl)-*N'*-éthylcarbonyldiimide comme agent d'activation sans *N*-hydroxysuccinimide et/ou sans *N*-hydroxysuccinamide dans un solvant organique.

18. Procédé de préparation d'un conjugué d'inhibiteur de la gyrase et d'albumine selon la revendication 1, comprenant la réaction d'un inhibiteur de la gyrase avec de l'albumine, **caractérisé en ce que** l'on fait réagir un inhibiteur de la gyrase et de l'albumine en présence de *N*-(3-diméthylaminopropyl)-*N'*-éthylcarbodiimide comme agent d'activation sans autre agent d'activation dans un solvant organique.

19. Procédé selon l'une des revendications 15 à 18, **caractérisé en ce que** le solvant organique est un solvant organique anhydre.

20. Procédé selon l'une des revendications 17 à 19, **caractérisé en ce que** l'on fait réagir l'inhibiteur de la gyrase avec du *N*-(3-diméthylaminopropyl)-*N'*-éthylcarbodiimide, on l'active en le chauffant et ensuite on fait réagir l'inhibiteur de la gyrase avec de l'albumine.

21. Procédé selon l'une des revendications 15 à 20, **caractérisé en ce que** l'albumine est la sérum albumine humaine.
